# EUROPÄISCHE PATENTSCHRIFT

(11) **EP 1 781 173 B1**
(45) Veröffentlichungstag und Bekanntmachung des Hinweises auf die Patenterteilung: **14.11.2012**
(21) Anmeldenummer: 05764074.0
(22) Anmeldetag: 07.07.2005
(51) Int. Cl.: A61B 5/151

(54) **VERFAHREN ZUR SELEKTIVEN STERILISATION VON DIAGNOSTISCHEN TESTELEMENTEN**
METHOD FOR THE SELECTIVE STERILISATION OF DIAGNOSTIC TEST ELEMENTS
PROCEDE DE STERILISATION SELECTIVE D'ELEMENTS DE TEST DIAGNOSTIQUE

(30) Priorität: 09.07.2004 DE 102004033219
(43) Veröffentlichungstag der Anmeldung: 09.05.2007
(73) Patentinhaber: Roche Diagnostics GmbH, 68305 Mannheim (DE); F.Hoffmann-La Roche AG, 4070 Basel (CH)
(72) Erfinder: SCHWIND, Karin, 67105 Schifferstadt (DE); FIEDLER, Wolfgang, 69514 Laudenbach (DE)
(86) Internationale Anmeldenummer: PCT/EP2005/007335
(87) Internationale Veröffentlichungsnummer: WO 2006/005503

(56) Entgegenhaltungen:
- EP-A- 1 402 812
- US-A1- 2004 106 941
- US-B1- 6 315 738

## Beschreibung

### [Technisches Gebiet]

Die vorliegende Erfindung betrifft ein Verfahren zur Herstellung von diagnostischen Testelementen für den Nachweis von Analyten in Körperflüssigkeiten.

### [Allgemeine Einleitung]

Die Untersuchung von Körperflüssigkeiten ermöglicht in der klinischen Diagnostik das frühzeitige und zuverlässige Erkennen von pathologischen Zuständen sowie die gezielte und fundierte Kontrolle von Körperzuständen. Für einzelne Analysen, die gezielt auf einen Parameter gerichtet sind, werden heutzutage oftmals wenige Mikroliter bis hin zu weniger als einem Mikroliter Blut benötigt. Zur Blutgewinnung wird üblicherweise durch die Haut z. B. in die Fingerbeere oder das Ohrläppchen der zu untersuchenden Person mit Hilfe einer sterilen, scharfen Lanzette gestoßen. Diese Methode eignet sich insbesondere, wenn die Analyse der Blutprobe unmittelbar nach der Blutgewinnung durchgeführt werden kann.

Für die chemische und biochemische Analyse von Körperflüssigkeiten haben sich in den dafür spezialisierten Labors, aber insbesondere auch für den Einsatz außerhalb fester Labors trägergebundene Schnelltests etabliert. Basierend auf einer eigens entwickelten Trockenchemie sind solche trägergebundenen Schnelltests, trotz der oftmals komplexen Reaktionen unter Beteiligung empfindlicher Reagenzien, einfach und unkompliziert, selbst von Laien, durchzuführen. Prominentestes Beispiel für trägergebundene Schnelltests sind Teststreifen für die Bestimmung des Blutglukosegehalts bei Diabetikern.

Bei heute verwendeten diagnostischen Testen für den Nachweis eines Analyts (z. B. Blutglukose) in einer Körperflüssigkeit (z. B. Blut) sind die Funktion des Stechens zum Erzeugen einer Hautöffnung und die Nachweisfünktion üblicherweise auf mehrere Bauteile aufgeteilt, z. B. eine Stechhilfe zum Stechen und Erzeugen eines Bluttropfens und ein analytisches Hilfsmittel, z. B. ein Teststreifen für die Aufnahme des Bluttropfens, die Weiterleitung des Blutes von der Aufnahmestelle zum Nachweisbereich, und den Nachweis eines Analyts, z. B. der Blutglukose.

Vor allem im Bereich des sogenannten "Home-Monitoring", also dort, wo medizinische Laien selbst einfache Analysen des Bluts durchführen, und dort insbesondere für die regelmäßige, mehrmals täglich durchzuführende Blutgewinnung durch Diabetiker für die Kontrolle der Blutglukosekonzentration, werden Lanzetten und dazu passende Geräte angeboten, die eine möglichst schmerzarme und verlässliche Blutgewinnung ermöglichen. Als Beispiele für Lanzetten und Stechhilfen seien die kommerziell erhältlichen Geräte (Stechhilfen) und Lanzetten Glucolet^{®} der Bayer AG und Softclix^{®} der Roche Diagnostics GmbH genannt. Solche Lanzetten und Geräte sind z. B. Gegenstand von WO 98/48695, EP 0,565,970, US 4,442,836 oder US 5,554,166.

Die eigenhändige Blutzuckerbestimmung ist heute eine weltweit verbreitete Methode in der Diabetes-Kontrolle. Blutzuckergeräte im Stand der Technik wie z. B. Accu-Chek Sensor^{®} (von Roche Diagnostics) bestehen aus einem Messgerät, in das ein Testelement (Teststreifen) eingeführt wird. Der Teststreifen wird mit einem Blutstropfen in Kontakt gebracht, der zuvor mittels einer Stechhilfe aus der Fingerbeere gewonnen wurde. Die zahlreichen Systemkomponenten (Lanzette, Stechhilfe, Teststreifen und Messgerät) benötigen viel Platz und bedingen ein relativ komplexes Handling. Inzwischen gibt es auch Systeme mit einem höheren Grad der Integration und damit einer einfacheren Handhabung. Hierzu gehören beispielsweise das AccuChek Compact^{®} (von Roche Diagnostics), das Glucometer Dex (von Bayer Diagnostics) sowie das Soft-Sense (von Medisense). Bei den beiden erstgenannten Systemen werden die Teststreifen im Messgerät magaziniert und für die Messung zur Verfügung gestellt.

Ein nächster Schritt der Miniaturisierung ist beispielsweise durch die Integration von mehreren Funktionen bzw. Funktionselementen in einem einzigen diagnostischen Testelement zu erreichen. Durch geeignete Kombination von Stechvorgang und sensorischer Analytkonzentrations-Erfassung in einer Baugruppe lässt sich beispielsweise der Bedienablauf deutlich vereinfachen. Solche Stech-Mess Disposables, im Folgenden auch integrierte Testelemente genannt, sind noch nicht auf dem Markt verfügbar, werden jedoch z. B. in DE 101 34 650, US 6,572,566, EP 0,199,484 und US 6,143,164 beschrieben.

### [Stand der Technik]

Bei Herstellung der eingangs erwähnten integrierten Testelemente, bei denen Stechbereich und Nachweisbereich in einer Baugruppe zusammengefasst sind, besteht grundsätzlich die Problematik, dass einerseits der Stechbereich steril sein muss, da er mit der Haut in Kontakt kommt bzw. in den Körper eindringt, und andererseits die empfindliche Nachweischemie im Nachweisbereich nicht durch den Herstellprozess geschädigt werden darf.

US 6,520,326 beschreibt beispielsweise die Sterilisierung eines integrierten Testelements indem der gesamte Sensor sterilisiert wird, insbesondere der Stech- und auch der Nachweisbereich. Die Schädigung der Nachweischemie im Nachweisbereich wird dabei durch eine spezielle Auswahl der Nachweischemie zu vermindern versucht.

In DE 101 42 232 werden mehrere zusammenhängende Lanzetten in Form eines Bandes gefertigt und parallel ein zweites Band, auf dem sich die Nachweisbereiche befinden, produziert. Das Lanzettenband wird sterilisiert, dann mit dem zweiten Band vereinigt und schließlich werden die Testelemente vereinzelt.

Das im Stand der Technik US 6,520,326 beschriebene Verfahren weist den Nachteil auf, dass bei der Auswahl der Nachweischemie eine Unempfindlichkeit gegen die Sterilisation gewährleistet werden muss. Es scheint fraglich, ob ein solches Verfahren überhaupt realisierbar ist, und inwieweit unter diesen Gegebenheiten eine ausreichende Performance, insbesondere hinsichtlich Empfindlichkeit und Reproduzierbarkeit zu erreichen ist.

Bei dem in DE 101 42 232 beschriebenem Herstellverfahren ist ein zusätzlicher Aufwand durch die Produktion zweier paralleler Bänder und durch das passgenaue Zusammenfügen von Stechbereich und Nachweisbereich notwendig.

Die Dokumente EP 1 402 812, US 6,315,738 und US 2004/106941 beschreiben jeweils die Herstellung eines integrierten Testelementes, bei dem zunächst das Nadelelement sterilisiert wird und erst anschließend eine Montage des Nachweiselementes erfolgt.

### [Aufgabe]

Aufgabe der vorliegenden Erfindung ist es, die Nachteile des Stands der Technik sowie das eingangs erwähnte Problem zu überwinden. Insbesondere ist es die Aufgabe der vorliegenden Erfindung, ein kompaktes, in hoher Stückzahl kostengünstig herstellbares integriertes diagnostisches Testelement bereitzustellen, bei dem der Stechbereich steril ist und die Nachweischemie im Nachweisbereich funktionstüchtig ist.

### [Beschreibung der Erfindung]

Die vorliegende Erfindung betrifft ein Verfahren zur Sterilisierung von integrierten Testelementen bestehend aus mindestens einem Nachweisbereich, der sterilisiert wird, und mindestens einem Stechbereich, der vor der Sterilisation geschützt wird.

Die integrierten Testelemente für den Nachweis eines Analyts in einer Körperflüssigkeit bestehen aus zwei Bereichen mit unterschiedlichen Anforderungen: dem Stechbereich zum Erzeugen einer Hautöffnung und dem Nachweisbereich. Der Stechbereich muss steril sein, um zu gewährleisten, dass während des Stechens keine Keime durch das Testelement in den Körper des Benutzers eindringen, während der Nachweisbereich nicht der Sterilisation ausgesetzt werden darf, da die Sterilisation die Nachweischemie, die sich im Nachweisbereich befinden, schädigt.

Im Zuge der Weiterentwicklung der Testsysteme hin zu mehr Bedienkomfort, geringerem Bedarf an Körperflüssigkeit für den Nachweis und einem schnelleren Nachweis versprechen integrierte Testelemente einen großen Fortschritt. Daraus ergibt sich ein hoher Zuwachs an Bedienkomfort und eine vereinfachte Handhabung, denn der Benutzer braucht lediglich das Testelement an einer Körperstelle zu platzieren und sich zu stechen. Der Analyt wird anschließend in den Nachweisbereich transportiert und dort gemessen.

Da bei integrierten Testelementen sterile und unsterile Bereiche auf einem Disposable integriert sind, wurde gefunden, dass es besonders vorteilhaft ist, die Testelemente möglichst vollständig zu produzieren und dann den unsterilen Bereich während der Sterilisation abzuschirmen. Dies vereinfacht den Herstellprozess insbesondere bei den hier erwähnten Stech - Mess Disposables.

Es sind grundsätzlich mehrere Verfahren zur Sterilisation von Stechbereichen, insbesondere Lanzetten aus Metall oder Kunststoff, bekannt. Eine Möglichkeit besteht in der Bestrahlung mit Gammastrahlung. Eine weitere Möglichkeit ist die Dampfsterilisation, z. B. mit Ethylenoxid (ETO). Es wurde gefunden, dass sowohl Gammastrahlung wie auch ETO-Sterilisation den Nachteil aufweisen, dass hierbei der Nachweisbereich nur mit großem Aufwand vor der Sterilisation und damit vor einer Inaktivierung geschützt werden kann. Um Gammastrahlung ausreichend abzuschirmen, müssen dicke Bleiplatten verwendet werden und selbst dann hat man das Problem der Beugung der Strahlung, was dazu führt dass auch hinter einer Abschirmung liegende Bauteile mit bestrahlt werden. Eine selektive Sterilisation, bei der der zu bestrahlende und der nicht zu bestrahlende Bereich sehr dicht nebeneinander liegen, wie dies insbesondere bei integrierten Testelementen der Fall ist, ist daher nur mit sehr großem Aufwand möglich. Für eine ETO-Sterilisation müsste einerseits der Nachweisbereich gasdicht verpackt werden und darüber hinaus der Sterilisationsraum gasdicht gegenüber der Umgebung abgedichtet werden, was insbesondere bei einer Fließbandproduktion nur unter sehr großem Aufwand möglich ist. Darüber hinaus lassen sich beide Verfahren schwerlich in eine Fließbandproduktion, wie sie für in hohen Stückzahlen produzierte Bauteile üblich ist, integrieren.

Es wurde gefunden, dass Elektronenstrahlung für die erfindungsgemäße selektive Sterilisation besonders geeignet ist, da sie sich einerseits für die Sterilisation metallischer Gegestände, wie zum Beispiel Lanzetten eignet, und sich andererseits relativ einfach abschirmen lässt, beispielsweise, um den Nachweisbereich zu schützen. So lässt sich sterilisierende Elektronenstrahlung bei entsprechender Dosis beispielsweise bereits durch ein 2 bis 5 mm starkes Metallblech, z. B. aus Stahl, Kupfer, Aluminium oder Blei, so gut wie vollständig, vorzugsweise um einen Faktor 1000, abschirmen, andererseits durchdringt die sterilisierende Strahlung mehrere Millimeter dicke Kunststoffschichten zu nahezu 100%. Darüber hinaus weist Elektronenstrahl-Sterilisation die Vorteile auf, dass sie trocken, schnell und bei niedriger Temperatur durchführbar ist, und leicht in eine Fließbandproduktion integriert werden kann. Somit können Test-elemente kostengünstig in großen Stückzahlen hergestellt werden.

Daher können für die selektive Sterilisation von diagnostischen Testelementen prinzipiell sämtliche bislang bekannten Varianten von Nachweisbereichen und Stechbereichen eingesetzt werden. Da der Nachweisbereich während der Sterilisation vor der Bestrahlung geschützt wird, brauchen der Nachweischemie keine zusätzlichen Stabilisatoren zugefügt zu werden. Diese Zusatzstoffe verringern in den meisten Fällen die Performance der Nachweischemie.

Darüber hinaus kann der Stechbereich insbesondere bereits vor der Sterilisation mit einem Schutz aus Kunststoff versehen werden, da die Elektronenstrahlung den Kunststoff ausreichend durchdringt und die darunterliegende Lanzette sterilisiert und der Kunststoff den Stechbereich vor erneuter Kontamination schützt. Das erfindungsgemäße Verfahren bietet den Vorteil, dass auf eine Produktion in einem Reinraum verzichtet werden kann, was Produktionsaufwand und -kosten deutlich reduziert.

Auf diese Weise kann selbst bei integrierten Testelementen, bei denen Stechbereich und Nachweisbereich in einer Baugruppe bzw. auf einem Bauteil zusammengefasst sind, während des Herstellprozesses der Stechbereich sterilisiert werden und gleichzeitig die Nachweischemie im Nachweisbereich vor der sterilisierenden Strahlung geschützt werden. Nach dem erfindungsgemäßen Verfahren hergestellte Testelemente können somit einfacher miniaturisiert werden und eignen sich daher besonders gut für den Einsatz in kompakten diagnostischen Testsystemen.

Selektive Sterilisation im Rahmen der vorliegenden Erfindung beschreibt Verfahren, bei denen gezielt bestimmte Bereiche eines Produkts, insbesondere der Stechbereich, sterilisiert werden und gleichzeitig andere definierte Bereiche des Produkts, insbesondere der Nachweisbereich, nicht sterilisiert werden.

Selektive Sterilisation bedeutet demnach eine räumliche Selektion im Sinne einer Auswahl zwischen bestrahlten und unbestrahlten Bereichen und bedeutet insbesondere nicht, dass die mit Elektronenstrahlung bestrahlten Bereiche nur selektiv, im Sinne von teilweise, sterilisiert werden, d.h. dass nur bestimmte Keime abgetötet werden.

Unter einem diagnostischen Testelement ist jede Form von trägergebundenen Schnelltests für die Diagnostik zu verstehen, insbesondere Schnelltests in Streifenform, sog. Teststreifen, hierbei insbesondere zur Bestimmung des Blutglukosegehalts bei Diabetikern.

Der Begriff Stechbereich umschreibt den Bereich des Testelements, mit dem eine Hautöffnung erzeugt wird, durch die Körperflüssigkeit, z. B. Blut oder interstitielle Flüssigkeit, austritt. Eine mögliche Ausführung eines Stechbereichs ist der vordere Teil einer Lanzette. Der Stechbereich ist in Kontakt bzw. in unmittelbarer Nähe zu der erzeugten Hautöffnung und muss steril sein, um zu verhindern, dass Keime vom Stechbereich in den Körper eindringen können. Der Austritt der Körperflüssigkeit kann ggf. durch unterstützende Maßnahmen, beispielsweise Erzeugen eines Unterdrucks, forciert werden.

Die Testzone beschreibt den Bereich, in dem sich die Nachweischemie befindet, mit der ein Analyt nachgewiesen wird. Innerhalb dieser Testzone befindet sich der Nachweisbereich. Mit Nachweisbereich ist der Bereich gemeint, in dem der Analyt auch tatsächlich zur Bestimmung der Konzentration gemessen wird. Da die Nachweischemie normalerweise durch Elektronenstrahlung geschädigt wird, muss während der selektiven Sterilisation sicher gestellt werden, dass der Nachweisbereich nicht der Elektronenstrahlung ausgesetzt wird. So kann zum Beispiel die Abschirmung so gewählt werden, dass zwar Teile der Testzone bestrahlt werden, z. B. um sicherzustellen, dass der Stechbereich und eventuell auch das Transportelement vollständig sterilisiert werden, der Nachweisbereich jedoch deutlich weniger bestrahlt wird. Vorzugsweise sollte die Abschirmung die sterilisierende Strahlung so gut wie vollständig, vorzugsweise um einen Faktor 1000, abschirmen.

Mit Analyt ist ein Bestandteil der Körperflüssigkeit gemeint, der im Nachweisbereich mit der Nachweischemie reagiert, so dass - ab einer gewissen Menge des Analyts - die Reaktion in einer Messanordnung gemessen werden kann. Eine bevorzugte Ausführungsform besteht darin, Blut als Probenflüssigkeit zu verwenden und Blutglukose als Analyt im Nachweisbereich nachzuweisen und daraus die Konzentration der Blutglukose zu ermitteln.

Neben Blut sind auch interstitielle Flüssigkeit und andere körpereigene Flüssigkeiten als Körperflüssigkeiten möglich. Ebenfalls ist es möglich, nicht nur einen Analyten, z. B. Blutglukose, sondern auch mehrere Analyten, z. B. HBA1C, nachzuweisen, und dies sowohl aus einer Körperflüssigkeit, z. B. Blut, als auch aus einem Gemisch mehrerer Körperflüssigkeiten, z. B. Blut plus interstitielle Flüssigkeit, durchzuführen.

Die eingesetzte Elektronenstrahlung ist, insbesondere in Bezug auf Wellenlänge bzw. Energiegehalt und Strahlendosis, so zu wählen, dass eine Sterilisierung des Stechbereiches der Testelemente sichergestellt ist.

Eine bevorzugte Ausführungsform des erfindungsgemäßen Verfahrens zur selektiven Sterilisation von Testelementen besteht darin, dass der Nachweisbereich gegen Elektronenstrahlung abgeschirmt ist, während das Testelement mit Elektronenstrahlung bestrahlt wird, wodurch der Stechbereich sterilisiert wird.

Während der Sterilisierung kann beispielsweise das gesamte Testelement inklusive der Abschirmung mit Elektronenstrahlung bestrahlt werden. In jedem Fall ist zu gewährleisten, dass der Stechbereich so umfassend bestrahlt wird, dass er nach dem Verfahrensschritt steril ist.

Unter Abschirmung des Nachweisbereichs ist jedes Mittel zu verstehen, das sicherstellt, dass der Nachweisbereich auf dem Testelement gegen die verwendete Elektronenstrahlung soweit geschützt ist, dass die im Nachweisbereich befindliche Nachweischemie nach dem Sterilisationsschritt funktionstüchtig ist. Eine bevorzugte Ausführung der Abschirmung sind Abschirmbleche oder Blenden, vorzugsweise aus Metall. Insbesondere sind Blei, Eisen, Kupfer und Aluminium sowie Legierungen daraus gut geeignete Abschirmmaterialien. Die Abschirmung kann sich beispielsweise dicht neben dem Transportband, auf dem die Testelemente gerichtet entlanggeführt werden, zwischen Elektronenstrahlquelle und Testelement befinden. Die Abschirmung kann aber auch nah an der Elektronenstrahlquelle angeordnet sein, insbesondere kann die Blende der Elektronenstrahlquelle dazu verwendet werden, dass die aus der Strahlungsquelle austretende Strahlung nur auf den zu sterilisierenden Bereich gelenkt wird, insbesondere auf den Stechbereich, während der zu schützende Bereich, insbesondere der Nachweisbereich, von der Strahlung abgeschirmt ist.

Eine weitere Ausführungsform besteht darin, dass sich die Abschirmung direkt an oder auf dem Testelement befindet und dort den Nachweisbereich abschirmt Vorzugsweise verhindert diese Abschirmung gleichzeitig den Austritt von Keimen aus dem unsterilen Nachweisbereich. Diese Ausführungsform bietet den zusätzlichen Vorteil, dass der Stechbereich nach der Sterilisation nicht durch einen Schutz gegen Kontamination durch den Nachweisbereich, z. B. über den Luftweg, versiegelt werden muss. Bei Sterilisation durch Elektronenstrahlung könnte diese Abschirmung des Nachweisbereichs eine Metall- bzw. Metall-Kunststoff-Folie sein, die z. B. über Verschweißen der Kunststoff-Folie mit dem Kunststoff des Testelements luftdicht aufgebracht wird. Anschließend wird beispielsweise das gesamte Testelement der sterilisierenden Strahlung ausgesetzt, - wobei es natürlich dennoch - auf Grund-der-lokalen Abschirmung des Nachweisbereichs - selektiv sterilisiert wird.

Eine weitere Ausführungsform besteht darin, den Nachweisbereich mit einem Schutz gegen Austreten von Keimen zu versehen, z. B. eine Kunststoff-Folie, die zwar verhindert, dass Keime aus unsterilen Bereichen, insbesondere dem Nachweisbereich über den Luftweg zum Stechbereich gelangen, die jedoch nicht, oder zumindest nicht in ausreichendem Masse, die sterilisierende Strahlung abschirmt. Dieser Schutz wird falls notwendig zumindest auf der Außenseite sterilisiert, vorzugsweise mit Plasma-Behandlung; danach werden Nachweisbereich und Schutz mit einer Abschirmung gegen Elektronenstrahlung, z. B. einem Metallblech, abgeschirmt, und der Stechbereich wird mit Elektronenstrahlung sterilisiert. Diese Ausführungsform bietet darüber hinaus den Vorteil, dass eventuell auf einen zusätzlichen Schutz des Stechbereichs verzichtet werden kann. Beispielsweise wird der Nachweisbereich auf den Testelementen mit einer Kunststoff-Folie verschweißt, und die Testelemente werden ohne zusätzlichen Stechbereichsschutz einzeln in einer Verpackung, z. B. einem Container, versiegelt

Die Elektronenstrahlen können von einer oder von mehreren Seiten auf das Testelement einwirken; entsprechend besteht die Abschirmung gegebenenfalls aus einem oder mehreren Teilen, um die Strahlung von allen erforderlichen Seiten abzuschirmen. Ist eine Bestrahlung von mehreren Seiten notwendig, können entweder mehrere Strahlungsquellen von verschiedenen Seiten gleichzeitig oder nacheinander auf das Testelement einwirken, oder eine Strahlungsquelle wirkt von mehreren Seiten auf das Testelement ein, oder das Testelement wird in dem Strahlengang so bewegt, z. B. gedreht oder mehrmals in unterschiedlicher Orientierung durch den Strahlengang geführt, dass der Stechbereich vollständig sterilisiert wird.

Die Bestrahlung mit Elektronenstrahlung kann so erfolgen, dass jedes Testelement einzeln abgeschirmt und sterilisiert wird, oder so, dass eine Anordnung mehrerer Testelemente abgeschirmt wird und anschließend die gesamte Anordnung bestrahlt wird.

Eine weitere Möglichkeit der Abschirmung besteht darin, dass die Testelemente in einer Verpackung angeordnet sind und die Verpackung so gestaltet ist, dass der Nachweisbereich der Testelemente gegen Elektronenstrahlung abgeschirmt wird und eine Bestrahlung der gesamten Verpackung erfolgt. Eine bevorzugte Ausführungsform der Verpackung stellt ein Container zur Magazinierung der Testelemente dar, z. B. eine Trommel, in der mehrere Testelemente einzeln in Kammern rotationssymmetrisch angeordnet sind, oder eine Box, in der die Testelemente gestapelt bzw. matrixähnlich angeordnet sind. Im Bereich des Nachweisbereichs ist in die Verpackung eine Abschirmung, z. B. ein Metallblech, integriert. Beispielsweise ist eine Kunststofftrommel im unteren Bereich mit Metall beschichtet. In einer weiteren bevorzugten Ausführungsform weist der Container selbst keine Abschirmung auf, sondern steht in einer Aufnahme, die als Abschirmung dient und in die entsprechende Vertiefungen angebracht wurden, beispielsweise eine Metallplatte mit runden Bohrungen, in die die Trommel genau so weit hineingesetzt wird, dass der Nachweisbereich der Testelemente in der Trommel von der Aufnahme abgeschirmt wird und gleichzeitig der Stechbereich bestrahlt werden kann.

Befinden sich die Testelemente während der Sterilisation in einer geschlossenen Verpackung, so ist die Verpackung vorzugsweise so zu gestalten, dass das Verpackungsmaterial, vorzugsweise Kunststoff und Pappe, für die eingesetzte Elektronenstrahlung durchlässig und gleichzeitig insoweit resistent gegen die Bestrahlung ist, dass es auch nach der Sterilisation die Anforderungen an die Verpackung, insbesondere Stabilität und Haltbarkeit des Aufdrucks, erfüllt.

Bei der zur selektiven Sterilisation eingesetzten Verpackung kann es sich sowohl bereits um die Endverpackung als auch um eine Primär- und Transportverpackung handeln, die während der Produktion dazu dient, die Testelemente von einem Prozessschritt zum nächsten zu transportieren.

Eine weitere Ausführungsform der Erfindung besteht darin, dass die Testelemente mit einer Zufuhrvorrichtung in den Strahlungsbereich einer Strahlungsquelle gebracht werden. Vorzugsweise beinhaltet die Zufuhrvorrichtung ein Transportband bzw. einen Gurt, auf dem die Testelemente, vorzugsweise in einer gerichteten Orientierung, angeordnet sind. Die Zufuhrvorrichtung transportiert die Testelemente in den Bereich der Sterilisation, z. B. eine Elektronenstrahlkammer oder einen Bestrahlungstunnel, und anschließend zum nächsten Produktionsschritt.

Eine weitere Möglichkeit des Verfahrens besteht darin, dass die Testelemente oder deren Verpackungen zusammenhängend gestaltet sind und die Testelementen durch Bewegung der zusammenhängenden Anordnung in den Strahlungsbereich gebracht werden. Zum Beispiel kann, wie in DE 101 42 232 beschrieben, das diagnostische Testelement aus Bandmaterial gefertigt werden. Hierbei werden zu einem Band bzw. Gurt zusammengefasste Stechbereiche, z. B. Lanzetten, sowie zu einem Band bzw. Gurt zusammengefasste Nachweisbereiche, z. B. Teststreifen, bereitgestellt Die beiden Bänder werden vereinigt und das Stechbereichsband mit dem Nachweisbereichsband verklebt. Nach der selektiven Sterilisation der Stechbereiche unter Abschirmung der Nachweisbereiche erfolgt eine Vereinzelung der miteinander verbundenen Bänder zu einzelnen Testelementen, beispielsweise durch Abschneiden der endständigen Testelemente.

In einer weiteren Ausführungsform werden die Nachweisbereiche direkt auf das Stechbereichsband aufgebracht, z. B. durch Aufdrucken. Anschließend werden die Nachweisbereiche abgeschirmt und das Testelementband selektiv sterilisiert und vereinzelt.

Ein Testelement, kann einen Stechbereich aufweisen, der zumindest in dem Bereich, der in die Haut eindringt, mit einem Schutz versiegelt ist, der ein Eindringen von Keimen verhindert. Mit Schutz ist hierbei jedes geeignete Mittel gemeint, das einerseits sicherstellt, dass der Stechbereich mit Elektronenstrahl sterilisiert werden kann und nach der Sterilisation steril bleibt, und andererseits ein unbeabsichtigtes Stechen des Benutzers bei der Handhabung des Testelements vermeidet. Eine bevorzugte Ausführungsformeines Stechbereichsschutzes stellt ein im Stand der Technik bekannter Schutz durch eine Kappe dar, wie er z. B. bei Softclix-Lanzetten vorhanden ist. Die Kappe umschließt den Stechbereich vollständig und verhindert somit ein Eindringen von Keimen. Vorzugsweise wird die Kappe bereits vor den Sterilisation auf den Stechbereich aufgebracht, z. B. angespritzt oder aufgesteckt. Um in der nachfolgenden Sterilisation sicherzustellen, dass eventuell im Stechbereich bzw. auf der Innenseite der Kappe vorhandene Keime unschädlich gemacht werden, ist es bei diesem Vorgehen essentiell, dass das Schutzmaterial für die sterilisierende Elektronenstrahlung ausreichend durchlässig ist.

Der Stechbereichsschutz kann aus einem durchstechbaren Material sein oder vor Gebrauch von dem Stechbereich abgenommen werden. Als durchstechbares Material kann insbesondere elastisches Material verwendet werden.

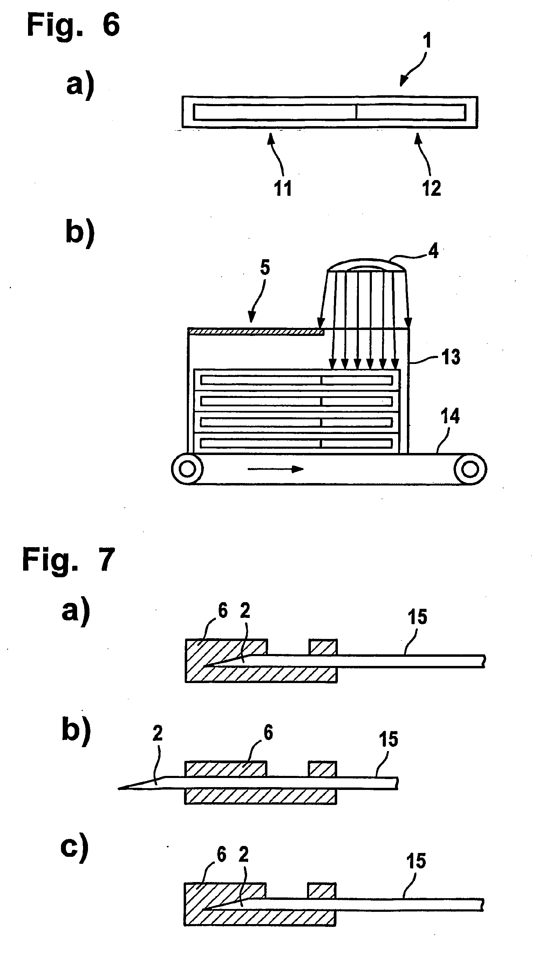

WO 01/66010 beschreibt beispielsweise eine Lanzette 15, die im Stechbereich (2), der hier mit Lanzettenspitze bezeichnet wird, von einem Stechbereichsschutz (6), einem sog. Lanzettenkörper, der aus einem elastischen Material gefertigt ist, vollständig umgeben ist (s. Fig. 7a). Während des Stechens durchstößt die Lanzettenspitze (2) den Stechbereichsschutz (6, Fig. 7b). Nach dem Stechen zieht sich die Lanzettenspitze (2) zurück und wird wieder vom Stechbereichsschutz (6) umschlossen (Fig. 7c). Eine derartige Ausführtungsform der Versiegelung des Stechbereichs kann vorteilhaft für integrierte Testelemente verwendet werden.

Der Schutz des Stechbereichs ist vorzugsweise so gestaltet, dass der Stechbereich nach der Benutzung wieder von dem Schutz umgeben ist. So können unbeabsichtigte Verletzungen des Benutzers oder anderer Personen vermieden werden.

Eine bevorzugte Ausführungsform beschreibt einen Stechbereich, z. B. eine Lanzettenspitze, der beim Stechen den Schutz, z. B. ein thermoplastisches Elastomer, durchstößt und sich nach dem Stechen wieder in den Schutz zurückzieht. In einer alternativen Ausführungsform wird der Schutz, z. B. eine Kunststoff-Kappe, vor dem Stechen manuell oder von dem Testgerät abgenommen. Nach Gebrauch wird der Schutz wieder auf den Stechbereich ausgesetzt oder das Testelement wird ohne Stechbereichsschutz entsorgt.

Ein optionales Merkmal der im Rahmen des erfindungsgemäßen Verfahrens herzustellenden Testelemente betrifft ein Transportelement das zwischen Stechbereich und Nachweisbereich angeordnet ist und mit dem der Analyt vom Stechbereich zum Nachweisbereich transportiert wird. Nachdem mit dem Stechbereich des Testelements eine Hautöffnung erzeugt wurde, wird eine Probe der Körperflüssigkeit z. B. Blut, gewonnen und der Analyt, z. B. Blutglukose, zum Nachweisbereich transportiert, um dort analysiert zu werden. Die Funktion des Transportelements besteht demnach darin, den Analyten oder auch mehrere Analyten - falls mehrere Analyten nachgewiesen werden sollen - vom Stechbereich zum Nachweisbereich zu transportieren. Üblicherweise wird als treibende Kraft für den Transport die Kapillarkraft eingesetzt. Für die Struktur des Transportelements sind verschiedene Ausführungsformen denkbar. Beispielsweise kann das Transportelement eine Kapillare aufweisen. Vorzugsweise beinhaltet das Transportelement einen Kapillarkanal bzw. einen Kapillarspalt, es kann aber auch eine Art Docht verwendet werden. Das Transportelement kann ein eigenes Bauteil sein, z. B. eine zusätzliche Kapillare, oder es kann in das Bauteil des Stechbereichs bzw. des Nachweisbereichs, beispielsweise in Form einer Nut in der Lanzette, oder als Kapillarkanal auf einem Teststreifen, integriert sein. Darüber hinaus kann das Transportelement selbstverständlich auch in ein anderes Bauteil des Testelements, insbesondere auch in den Schutz des Stechbereichs, integriert sein.

Bei einem erfindungsgemäßen Testelement kann der Bedarf an Körperflüssigkeit für den Nachweis eines Analyts deutlich reduziert werden, denn durch die Integration von Stechen und Nachweis auf einem Bauteil kann die Transportstrecke vom Stech- zum Nachweisbereich deutlich verkürzt werden. Da ein Großteil der Körperflüssigkeit für die Benetzung der Transportstrecke und nur ein geringer Teil für den tatsächlichen Nachweis eines Analyts benötigt wird, ergibt sich durch die Verkürzung der Transportstrecke eine Verringerung der benötigten Körperflüssigkeit. Ein weiterer Vorteil der Verkürzung der Transportstrecke besteht darin, dass die zu untersuchende Körperflüssigkeit bei gleicher Fließgeschwindigkeit, z. B. auf Grund von Kapillarkräften, schneller im Nachweisbereich ist, wodurch der gesamte Prozess zum Nachweis eines Analyts beschleunigt wird.

Die vorliegende Erfindung umfasst ein Verfahrens zur Herstellung eines diagnostischen Testelements gemäß den erwähnten Merkmalen mittels selektiver Sterilisation.

### [Beschreibung der Figuren]

Die Erfindung wird durch die nachfolgenden Figuren näher erläutert:
- Fig: 1: Selektive Sterilisation eines diagnostischen Testelements mit Stechbereichsschutz.
- Fig. 2: Selektive Sterilisation eines diagnostischen Testelements mit teilweise bestrahlter Testzone.
- Fig. 3: Diagnostisches Testelement mit Keimschutz.
- Fig. 4: Selektive Sterilisation von zusammenhängenden diagnostischen Testelementen.
- Fig. 5: Selektive Sterilisation von diagnostischen Testelementen in einer Verpackung.
- Fig. 6: Prinzipielles Vorgehen bei der selektiven Sterilisation eines diagnostischen Testelements.
- Fig. 7: Stechbereichsschutz aus durchstechbarem Material.
- Fig. 8: Anwendung der selektiven Sterilisation auf ein Stech-Mess Disposable mit einer sog. pooling area.
- Fig. 9: Anwendung der selektiven Sterilisation auf ein diagnostisches Testelement mit einem ringförmigen Karillarspalt.
- Fig. 10: Anwendung der selektiven Sterilisation auf ein diagnostisches Testelement mit einer flexiblen Sammelfolie.
- Fig. 11: Anwendung der selektiven Sterilisation auf ein diagnostisches Testelement mit manuell abnehmbarem Stechbereichsschutz und gegen den Testelement-Grundkörper verschieblicher Lanzette.

Die Ziffern in den Figuren bedeuten:
1 Diagnostisches Testelement
2 Stechbereich
3 Nachweisbereich
4 Sterilisierende Elektronenstrahlung
5 Abschirmung des Nachweisbereichs
6 Schutz des Stechbereichs
7 Testzone
8 Keimschutz
9 Primärverpackung für Testelemente
10 Aufnahme für Primärverpackung
11 Zu schützender Teil
12 Zu bestrahlender Teil
13 Umverpackung
14 Transportband
15 Lanzette
16 Pooling area
17 Kapillarkanal bzw. -spalt
18 Unterkanäle
19 Sammelfolie
20 Haut
21 Hautöffnung
22 Testelement-Grundkörper

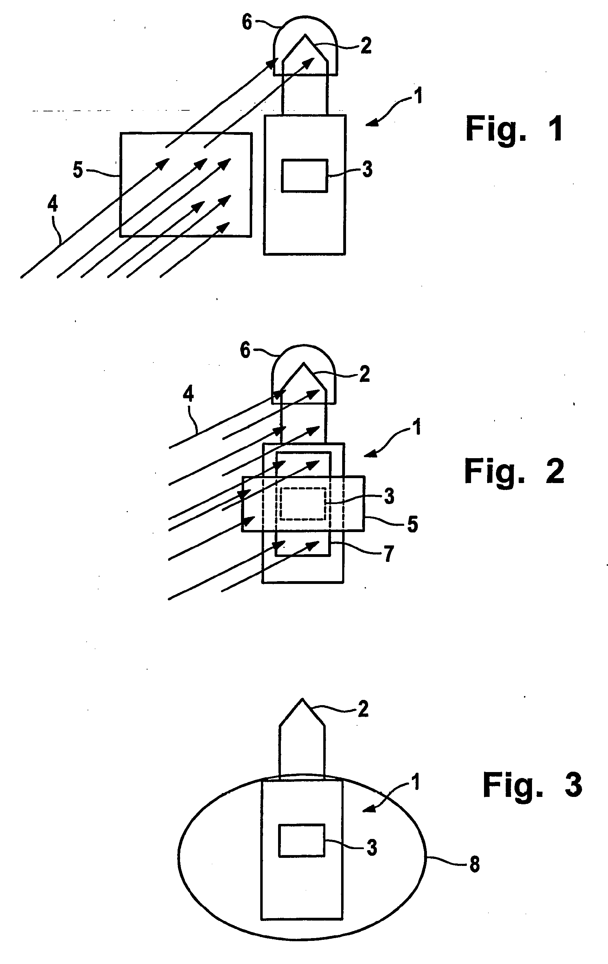

In Fig. 1 ist schematisch eine Ausführungsform des Verfahrens zur selektiven Sterilisation abgebildet. Die Figur zeigt ein diagnostisches Testelement (1) mit einem Stechbereich (2) und einem Nachweisbereich (3), wobei das Testelement (1) durch Elektronenstrahlung (4) sterilisiert wird. Hierbei schirmt die Abschirmung (5) den Nachweisbereich (3) so ab, dass der Nachweisbereich (3) nicht sterilisiert wird, um zu verhindern, dass die im Nachweisbereich (3) vorhandene Nachweischemie, insbesondere Enzyme, durch die Sterilisation geschädigt wird. Der Stechbereich (2) ist mit einem Schutz (6) versiegelt, der ein Eindringen von Keimen verhindert. Der Schutz (6) ist für die sterilisierende Elektronenstrahlung (4) durchlässig und lässt daher eine Sterilisation des Stechbereichs (2) auch bei aufgesetztem Schutz (6) zu und stellt gleichzeitig sicher, dass der Stechbereich (2) nach der Sterilisation bis zum bestimmungsgemäßen Gebrauch steril bleibt.

Fig. 2 zeigt eine weitere Ausführungsform des erfindungsgemäßen Verfahrens. Hierbei ist die Testzone (7), auf der sich die Nachweischemie befindet, deutlich größer als der Nachweisbereich (3), der den Bereich umschreibt, in dem der Analyt tatsächlich nachgewiesen wird. Die Abschirmung (5) deckt den Nachweisbereich (3) vollständig ab, die Testzone (7) wird jedoch nur teilweise abgeschirmt. Der Stechbereich (2) ist vollständig mit einem Schutz (6) versiegelt, der ein Eindringen von Keimen verhindert. Während der anschließenden Sterilisation mit Elektronenstrahlung (4) werden entsprechend außer dem Stechbereich (2) auch die Nachweischemie, die sich in dem ungeschützten Bereich der Testzone befindet, bestrahlt. Die dadurch auftretende Schädigung der Nachweischemie in diesem Bereich ist jedoch für den Nachweis des Analyten unerheblich, da für die Messung ausschließlich die Reaktion des Analyten mit der Nachweischemie im Nachweisbereich (3) berücksichtigt wird.

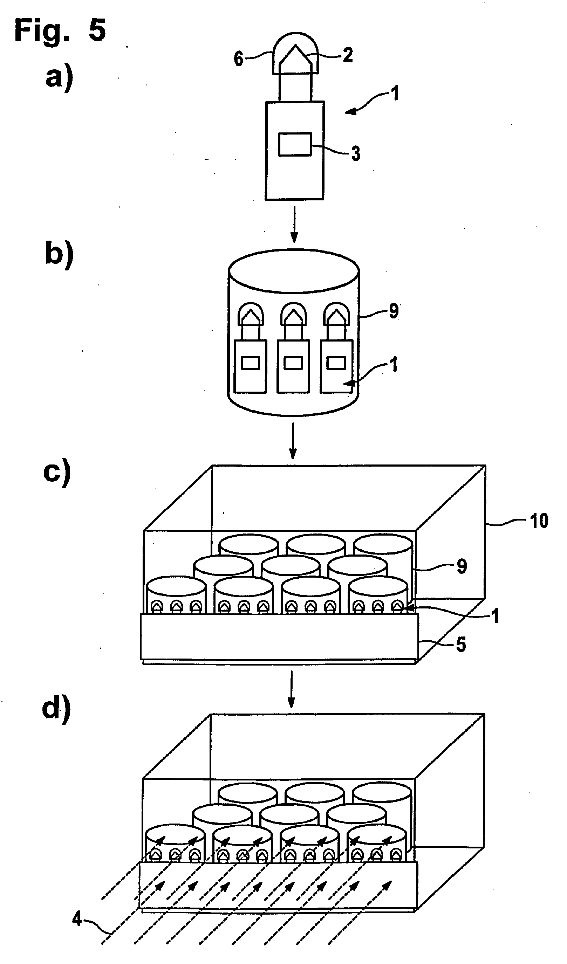

Fig. 3 zeigt eine weitere Ausführungsform des erfindungsgemäßen Verfahrens. Hierbei ist der Nachweisbereich (3) mit einem Schutz gegen Austreten von Keimen (8) versehen und dafür der Stechbereich (2) nicht geschützt. Anschließend wird das Testelement (1) beispielsweise anaolg Fig. 5b-d in eine Primärverpackung (9) gestellt, die Nachweisbereiche abgeschirmt und die Stechbereiche bestrahlt. Bevorzugter Weise sind die Testelemente innerhalb der Primärverpackung einzeln verpackt, z. B. in einzelne Kammern innerhalb der Verpackung gelegt oder einzeln verschweißt, so dass bei der Entnahme eines Testelements die Sterilität der übrigen Testelemente gewährleistet ist.

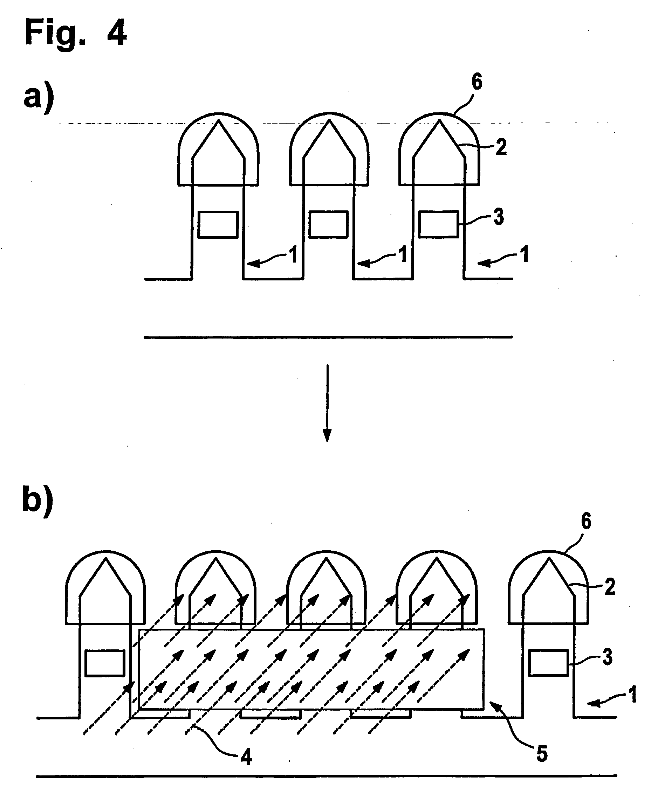

Fig. 4 zeigt eine weitere Ausführungsform des erfindungsgemäßen Verfahrens. In Fig. 4a sind zusammenhängend gestaltete Testelemente (1) mit Stech- (2) und Nachweisbereich (3) dargestellt, wobei der Stechbereich (2) mit einem Schutz (6) versehen ist. Fig. 4b zeigt die in Fig. 4a abgebildete Anordnung während der selektiven Sterilisation. Die Elektronenstrahlung (4) bestrahlt die zusammenhängenden Testelemente (1), wobei der jeweilige Nachweisbereich (3) durch eine Abschirmung (5) vor der Bestrahlung (4) geschützt wird.

Fig. 5 zeigt eine weitere Ausführungsform des erfindungsgemäßen Verfahrens. In Fig. 5a ist ein Testelement (1) mit Stechbereich (2), Nachweisbereich (3) sowie Schutz des Stechbereichs (6) abgebildet, das in Fig. 5b zusammen mit mehreren Testelementen in eine Primärverpackung (9), z. B. einen Container oder eine Trommel, gerichtet verpackt wird. Anschließend werden mehrere Primärverpackungen (9) in Fig. 5c in eine Aufnahme (10) gestellt, die eine Abschirmung (5) aufweist, die so gestaltet ist, dass in der anschließenden Bestrahlung mit Elektronenstrahlung (4) gemäß Fig. 5d alle Nachweisbereiche (3) vor der Strahlung (4) geschützt sind und gleichzeitig alle Stechbereiche (2) durch die Elektronenstrahlung (4) sterilisiert werden.

Fig. 6 verdeutlicht das Prinzip der selektiven Sterilisation. Fig. 6a zeigt ein Testelement (1), das einen zu schützenden Teil (11) aufweist, der nicht bestrahlt werden soll, und einen zu bestrahlenden Teil (12), der durch die Elektronenstrahlung sterilisiert werden soll. In Fig. 6b ist ein prinzipielles Beispiel für den Bestrahlungsprozess in einer Elektronenstrahl-Anlage gezeigt. Die Strahlung (4) bestrahlt den zu bestrahlenden Teil (12) einer Anordnung von Testelementen (1), die sich in einer Umverpackung (13) befinden, während der zu schützende Teil (11) durch die Abschirmung (5) abgeschirmt wird. Die Testelemente werden auf einem Transportband (14) in den Strahlungsbereich gebracht. Die Abschirmung (5) kann sowohl an dem Band (14) oder an der Umverpackung (13) befestigt sein.

Die folgenden Figuren illustrieren die breite Anwendbarkeit des erfindungsgemäßen Verfahrens auf verschiedenartige integrierte Testelemente.

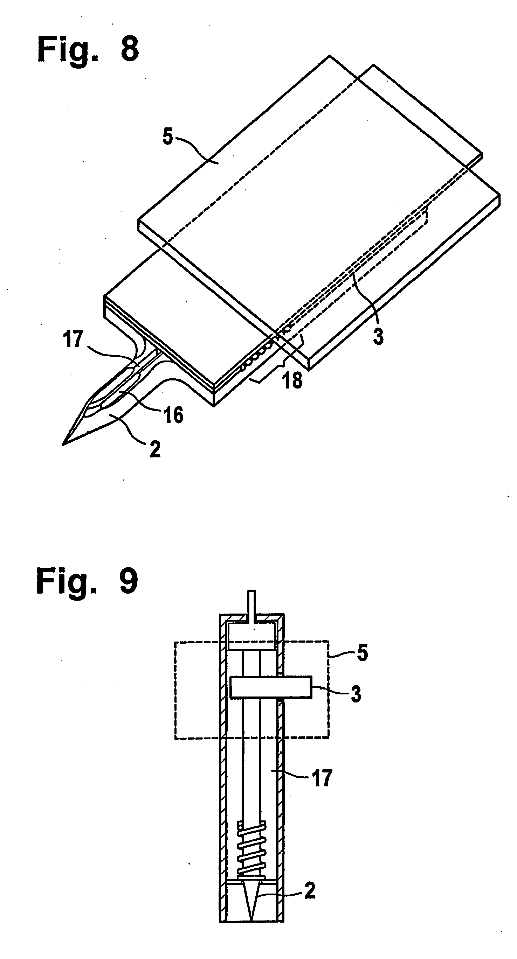

Fig. 8 zeigt die Anwendung des erfindungsgemäßen Verfahrens auf ein Stech-Mess Disposable. Die Körperflüssigkeit wird hierbei in einer sog. pooling area (16) während des Stechens mit dem Stechbereich (2) gesammelt und über Kapillarkanal (17) und Unterkanäle (18) in den Nachweisbereich (3) transportiert. Der Nachweisbereich (3) wird während der selektiven Sterilisation von einer darüber liegenden Abschirmung (5) abgeschirmt.

Fig. 9 zeigt eine weitere mögliche Anwendung des erfindungsgemäßen Verfahrens. Während des Stechens wird die Körperflüssigkeit hierbei in einem ringförmigen Kapillarspalt (17) vom Stechbereich (2) zum Nachweisbereich (3) transportiert. Der Nachweisbereich (3) wird während der selektiven Sterilisation von der Abschirmung (5) abgeschirmt.

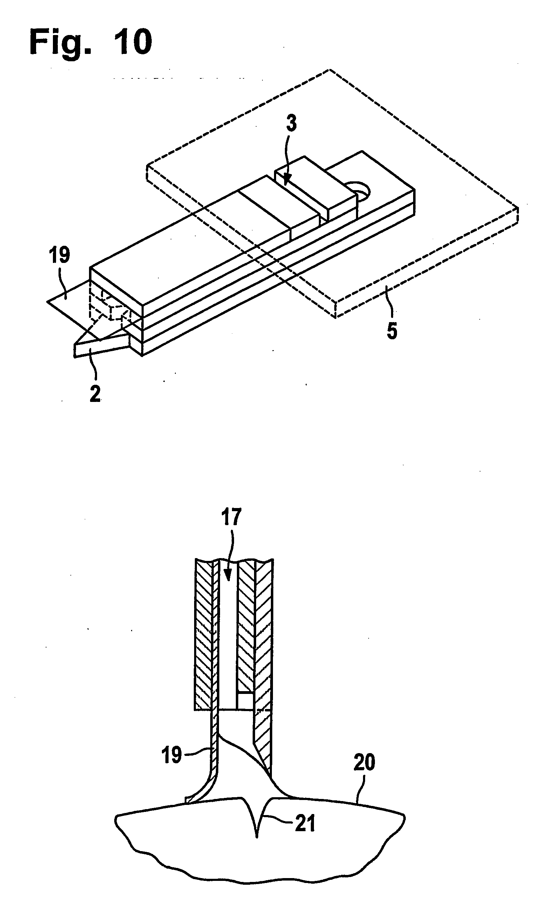

Fig. 10 zeigt eine weitere mögliche Anwendung des erfindungsgemäßen Verfahrens. Das integrierte Testelement weist neben dem Stechbereich (2) eine hydrophile Sammelfolie (19) auf, die frei über dem Stechbereich hängt und sich während des Stechens zur Seite biegt und an die Haut (20) anlegt. Nach Zurückziehen des Stechbereichs (2) aus der Haut (20) wird die Körperflüssigkeit aus der Hautöffnung (21) entlang der Sammelfolie (19) in den Kapillarkanal (17) und von dort weiter in den Nachweisbereich (3) transportiert. Der Nachweisbereich (3) wird während der selektiven Sterilisation von der Abschirmung (5) abgeschirmt.

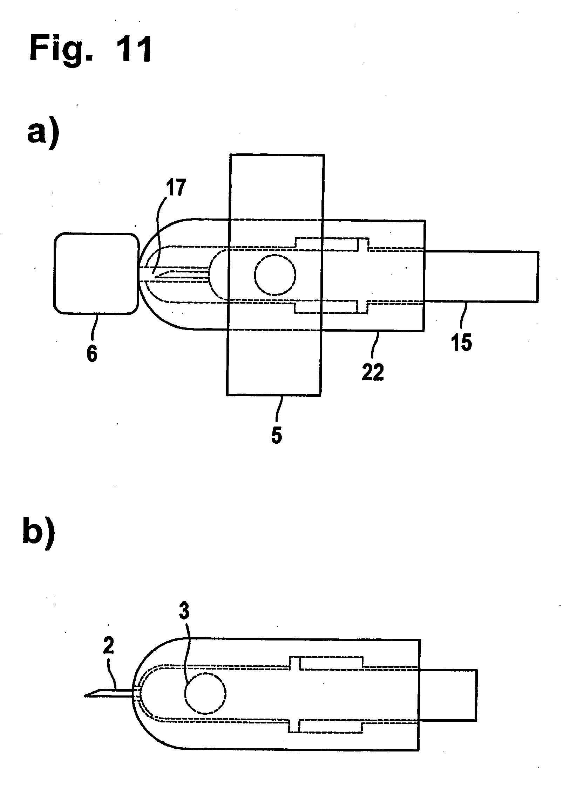

Fig. 11 zeigt eine weitere mögliche Anwendung des erfindungsgemäßen Verfahrens. Die Schutzkappe (6) schützt den Stechbereich (2) gegen Kontamination und wird vor Gebrauch manuell abgenommen (Fig. 11a). Die Lanzette (15) mit dem Stechbereich (2) verschiebt sich während des Stechens gegenüber dem Testelement-Grundkörper (22) (Fig. 11b) und zieht sich anschließend wieder in den Grundkörper zurück, wobei die Körperflüssigkeit durch den Kapillarkanal (17) zum Nachweisbereich (3) gelangt. Der Nachweisbereich (3) wird während der selektiven Sterilisation im Herstellprozess von der Abschirmung (5) abgeschirmt.

## Patentansprüche

1. Verfahren zur Herstellung integrierter, diagnostischer Testelemente (1), die einen Stechbereich (2) zum Erzeugen einer Hautöffnung, sowie einen Nachweisbereich (3) zum Nachweis eines Analyten in einer Körperflüssigkeit aufweisen, wobei das Verfahren die Schritte
- Abschirmung des Nachweisbereiches (3) gegen Elektronenstrahlung (4) und anschließend
- Bestrahlung des Testelementes (1) mit Elektronenstrahlung (4)
beinhaltet und wobei der Stechbereich (2), nicht jedoch der Nachweisbereich (3) durch Elektronenstrahlung (4) sterilisiert wird.

2. Verfahren gemäß Anspruch 1, bei dem eine Anordnung mehrerer Testelemente (1) durch Abschirmungsmittel abgeschirmt wird und eine Bestrahlung der gesamten Anordnung erfolgt.

3. Verfahren gemäß Anspruch 1, bei dem Testelemente (1) in einer Verpackung angeordnet sind und die Verpackung (9) oder eine Aufnahme für die Verpackung (10) so gestaltet ist, dass der Nachweisbereich (3) der Testelemente (1) gegen Elektronenstrahlung (4) abgeschirmt wird und eine Bestrahlung der gesamten Verpackung (9) erfolgt.

4. Verfahren gemäß einem der Ansprüche 1, 2 oder 3, bei dem der Nachweisbereich (3) mit einem Schutz gegen Austreten von Keimen versehen ist und der Stechbereich (2) nicht mit einem Schutz gegen Eindringen von Keimen versehen ist.

5. Verfahren gemäß Anspruch 1, bei dem Testelemente (1) mit einer Zufuhrvorrichtung in den Strahlungsbereich einer Strahlungsquelle gebracht werden.

6. Verfahren gemäß Anspruch 5, bei dem die Zufuhrvorrichtung ein Transportband aufweist, auf dem die Testelemente (1) angeordnet sind.

7. Verfahren gemäß einem der Ansprüche 3 oder 5, bei dem die Testelemente (1) oder deren Verpackungen (9) zusammenhängend gestaltet sind und die Testelemente (1) durch Bewegung der zusammenhängenden Anordnung in den Strahlungsbereich gebracht werden.

8. Verfahren gemäß Anspruch 1, bei dem der Stechbereich (2) der Testelemente (1) zumindest in einem Bereich, der in die Haut eindringt, mit einem Schutz (6) versiegelt ist, der ein Eindringen vom Keimen verhindert.

9. Verfahren gemäß Anspruch 8, bei dem der Schutz (6) aus einem durchstechbaren Material ist oder der Schutz (6) von dem Stechbereich (2) abgenommen werden kann.

10. Verfahren gemäß Anspruch 1, bei dem das Testelement (1) zwischen Stechbereich (2) und Nachweisbereich (3) ein Transportelement aufweist, womit der Analyt vom Stechbereich (2) zum Nachweisbereich (3) transportiert wird.

## Claims

1. Process for producing integrated, diagnostic test elements (1) comprising a lancing area (2) to generate a skin opening as well as a detection area (3) to detect an analyte in a body fluid, wherein the process comprises the steps
- screening the detection area (3) from electron radiation (4) and subsequently
- irradiating the test element (1) with electron radiation (4) and
wherein the lancing area (2), but not the detection area (3) is sterilized by electron radiation.

2. Process according to claim 1, in which an arrangement of several test elements (1) is screened by screening means and the entire arrangement is irradiated.

3. Process according to claim 1, in which the test elements (1) are arranged in a package and the package (9) or a holder for the package (10) is designed such that the detection area (3) of the test elements (1) is screened against electron radiation (4) and the entire package (9) is irradiated.

4. Process according to one of the claims 1, 2 or 3, in which the detection area (3) is provided with a protection against the escape of germs and the lancing area (2) is not provided with a protection against penetration by germs.

5. Process according to claim 1, in which test elements (1) are brought into the radiation area of a radiation source by a feeding device.

6. Process according to claim 5, in which the feeding device has a transport tape on which the test elements (1) are arranged.

7. Process according to one of the claims 3 or 5, in which the test elements (1) or their packages (9) are designed to be connected and the test elements (1) are brought into the radiation area by moving the connected arrangement.

8. Process according to claim 1, in which at least one region of the lancing area (2) of the test elements (1) which penetrates the skin is sealed with a protection (6) that prevents a penetration of germs.

9. Process according to claim 8, in which the protection (6) is made of a material that can be pierced or the protection (6) can be removed from the lancing area (2).

10. Process according to claim 1, in which the test element (1) has a transport element between the lancing area (2) and the detection area (3) by means of which the analyte is transported from the lancing area (2) to the detection area (3).

## Revendications

1. Procédé de fabrication d'éléments de test diagnostique intégrés (1), comprenant une zone de piqûre (2) servant à créer une ouverture dans la peau, ainsi qu'une zone de décèlement (3) servant à déceler un analyte dans un liquide organique, procédé comportant les étapes consistant à
- protéger la zone de décèlement (3) contre le faisceau d'électrons (4)
puis à
- exposer l'élément de test (1) au faisceau d'électrons (4),
et dans lequel la zone de piqûre (2), mais pas la zone de décèlement (3) est stérilisée par faisceau d'électrons (4).

2. Procédé selon la revendication 1, dans lequel un ensemble de plusieurs éléments de test (1) est protégé par des moyens formant écran, et l'ensemble complet est exposé au rayonnement.

3. Procédé selon la revendication 1, dans lequel les éléments de test (1) sont placés dans un emballage, et l'emballage (9) ou un logement pour l'emballage (10) est conçu de façon à protéger la zone de décèlement (3) des éléments de test (1) contre le faisceau d'électrons (4) et à exposer l'ensemble de l'emballage (9) au rayonnement.

4. Procédé selon l'une des revendications 1, 2 ou 3, dans lequel la zone de décèlement (3) est munie d'une protection contre un échappement de germes, et la zone de piqûre (2) est dépourvue d'une protection contre une pénétration de germes.

5. Procédé selon la revendication 1, dans lequel les éléments de test (1) sont amenés par un dispositif d'alimentation dans la zone de rayonnement d'une source de rayonnement.

6. Procédé selon la revendication 5, dans lequel le dispositif d'alimentation comprend une bande transporteuse sur laquelle sont placés les éléments de test (1).

7. Procédé selon l'une des revendications 3 ou 5, dans lequel les éléments de test (1) ou leurs emballages (9) sont disposés de façon contiguë, et les éléments de test (1) sont amenés dans la zone de rayonnement par un mouvement de l'ensemble contigu.

8. Procédé selon la revendication 1, dans lequel la zone de piqûre (2) des éléments de test (1), au moins sur une partie qui pénètre dans la peau, est scellée par une protection (6) qui empêche une pénétration de germes.

9. Procédé selon la revendication 8, dans lequel la protection (6) est réalisée en un matériau transperçable, ou la protection (6) peut être ôtée de la zone de piqûre (2).

10. Procédé selon la revendication 1, dans lequel l'élément de test (1) comporte un élément de transport entre zone de piqûre (2) et zone de décèlement (3), au moyen duquel l'analyte est transporté de la zone de piqûre (2) vers la zone de décèlement (3).
